# EUROPEAN PATENT APPLICATION

(11) **EP 4 459 283 A1**
(43) Date of publication of application: **06.11.2024**
(21) Application number: 23766299.4
(22) Date of filing: 12.01.2023
(51) Int. Cl.: G01N 33/543, G01N 21/05, G01N 21/78

(54) **TEST STRIP**

(30) Priority: 07.03.2022 JP 2022034379
(71) Applicant: Terumo Kabushiki Kaisha, Tokyo 151-0072 (JP)
(72) Inventor: AIKAWA, Ryokei, Ashigarakami-gun, Kanagawa 259-0151 (JP)
(74) Representative: TBK
(86) International application number: PCT/JP2023/000606
(87) International publication number: WO 2023/171118

(57) **Abstract**

A test strip (10A) includes a first reagent portion (76a) and a second reagent portion (76b) provided in a flow path (30) so as to face each other while being separated from each other. The flow path (30) includes a first step portion (84) located at an upstream end of the flow path (30) in the first reagent portion (76a), and a second step portion (86) located at an upstream end of the flow path (30) in the second reagent portion (76b). In a sample flow direction in the flow path (30), the first step portion (84) and the second step portion (86) are offset from each other.

## Description

### Technical Field

The present invention relates to a test strip.

### Background Art

WO 2021/192952 A discloses a test strip including a flow path for circulating a sample (blood) introduced from an inlet portion by capillary force. In the flow path, a reagent portion is arranged so as to form a lower surface of the flow path. An upper surface of the flow path is located above the reagent portion.

### Summary of Invention

In the above-described test strip, when the sample introduced from the inlet portion reaches the reagent portion, the reagent component of the reagent portion diffuses toward the sample. In this case, since the diffusion distance of the reagent component is a distance from the reagent portion to the upper surface of the flow path, the diffusion time of the reagent component becomes relatively long. Therefore, the time (measurement standby time) from introduction of the sample into the inlet portion to completion of diffusion of the reagent component into the sample becomes long.

An object of the present invention is to solve the above problem.

One aspect of the present invention is a test strip having a flow path for circulating a sample introduced from an inlet portion by capillary force, the test strip including a first reagent portion and a second reagent portion provided in the flow path so as to face each other while being separated from each other. The flow path includes a first step portion located at an upstream end of the flow path in the first reagent portion and a second step portion located at an upstream end of the flow path in the second reagent portion, and the first step portion and the second step portion are offset from each other in a flow direction of the sample in the flow path.

According to the present invention, the first reagent portion and the second reagent portion are provided in the flow path so as to face each other while being separated from each other. Therefore, when the sample introduced from the inlet portion arrives between the first reagent portion and the second reagent portion (reaction flow path), the reagent component of the first reagent portion and the reagent component of the second reagent portion are diffused into the sample. At this time, the diffusion distance of the reagent component is half the distance between the first reagent portion and the second reagent portion. Therefore, the diffusion time of the reagent component into the blood can be made relatively short.

In addition, the first step portion and the second step portion are offset from each other in the flow direction of the sample in the flow path. In this case, the sample passes through one of the first step portion and the second step portion and then passes through the other of the first step portion and the second step portion. As a result, the flow path resistance can be dispersed in the flow direction of the sample in the flow path as compared with a case where the first step portion and the second step portion are provided at the same position in the flow direction of the sample in the flow path. Therefore, the sample introduced from the inlet portion can be smoothly guided to the reaction flow path. Therefore, the measurement standby time can be shortened.

### Brief Description of Drawings

Fig. 1 is a plan view illustrating an overall configuration of a component measurement system including a test strip according to a first embodiment of the present invention.
Fig. 2 is a perspective view of the test strip of Fig. 1.
Fig. 3 is an exploded perspective view of the test strip of Fig. 2.
Fig. 4 is a longitudinal sectional view of the test strip of Fig. 2.
Fig. 5 is an enlarged cross-sectional view of Fig. 4.
Fig. 6 is a flowchart illustrating a method of manufacturing the test strip of Fig. 2.
Fig. 7 is a partially omitted cross-sectional explanatory view of the component measurement system of Fig. 1.
Fig. 8 is an enlarged cross-sectional view of a test strip according to a second embodiment of the present invention.
Fig. 9 is an experimental result for explaining the effect of the test strip according to the present invention.
Fig. 10 is an enlarged cross-sectional view of a test strip according to a comparative example.

### Description of Embodiments

### (First embodiment)

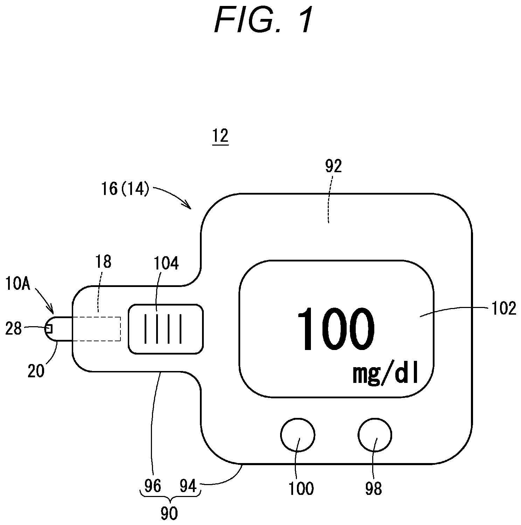

As illustrated in Fig. 1, a component measurement system 12 according to a first embodiment of the present invention includes a test strip 10A capable of holding a sample, and a component measurement device 14 that measures the amount of an analyte contained in the sample by attaching the test strip 10A.

A sample is introduced into the test strip 10A. The test strip 10A is configured to be held at a detection target position in the component measurement device 14 in a state in which a sample and a reagent are reacted with each other to cause coloring (colored state). On the other hand, the component measurement device 14 optically detects a reaction product between the sample and the reagent at the detection target position of the test strip 10A. Note that the test strip 10A is also referred to as a chip, a sensor, or the like. The "sample" may be whole blood (blood) or separated plasma. In addition, the sample may be another body fluid or an aqueous solution containing an analyte.

Hereinafter, the component measurement system 12 (blood glucose level measurement system) that detects the amount of an analyte (glucose in this case) when the sample is blood will be representatively described. In particular, the component measurement device 14 is configured as a blood glucose meter 16 that measures the blood glucose level by including a measurement unit 18 that irradiates the detection target position with measurement light of a predetermined wavelength and detects the measurement light (transmitted light) transmitted through the detection target.

The test strip 10A includes a reagent. The reagent contains a coloring reagent that is dissolved in the sample and reacts according to the amount of the analyte in the sample. Therefore, when the reagent and the analyte come into contact with each other, a color reaction occurs in which the coloring reagent develops color, and a color component (reaction product) is generated.

The reagent of the present embodiment specifically reacts with glucose. Examples of the reagent of the present embodiment include: a mixed reagent of (i) glucose oxidase (GOD), (ii) peroxidase (POD), (iii) 1-(4-sulfophenyl)-2,3-dimethyl-4-amino-5-pyrazolone, and (iv) N-ethyl-N-(2-hydroxy-3-sulfopropyl)-3,5-dimethylaniline sodium salt monohydrate (MAOS); and a mixed reagent of glucose dehydrogenase (GDH) and a tetrazolium salt. In addition, the reagent of the present embodiment contains a hemolytic agent and a hydrophilizing agent (sugar alcohol or the like). The reagent may further contain a buffer such as a phosphate buffer, a mediator, and an additive.

The type and components of the reagent are not limited to the above. In the present embodiment, the blood glucose meter 16 detects a mixture of a color component (reaction product) and a sample. In particular, when the detection target position is irradiated with measurement light having a predetermined wavelength and the measurement light (transmitted light) transmitted through the detection target is detected, the prepared mixed reagent solution is preferably directly applied to a predetermined position in the test strip 10A without using a porous member or a carrier and dried.

In addition, the component measurement system 12 is used as a personal use measurement system operated by a user (patient). For example, the user uses the test strip 10A and the blood glucose meter 16 to measure his or her blood glucose level and control his or her blood glucose level. Note that the component measurement system 12 may be used in a medical facility or the like as a device by which a medical worker measures a blood glucose level of a patient.

When the test strip 10A is attached to the blood glucose meter 16, a part of the test strip 10A protrudes to the outside of the blood glucose meter 16. The test strip 10A has an opening (inlet portion 28) on its end surface. The blood glucose level is measured by the blood glucose meter 16 by introducing blood into the test strip 10A via the inlet portion 28. The test strip 10A is configured as a disposable component that is discarded after each measurement.

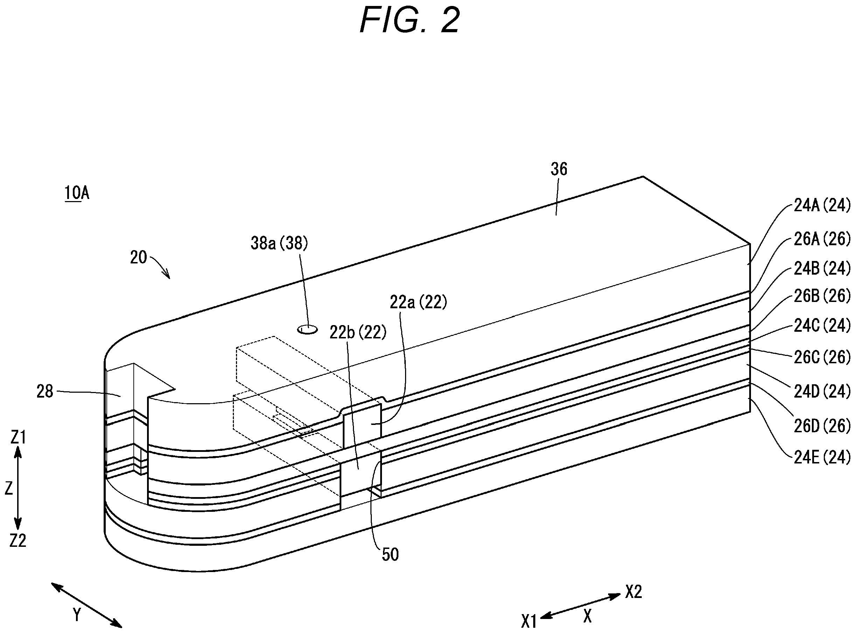

As illustrated in Fig. 2, the test strip 10A includes a main body portion 20 having a test paper shape (flat plate shape), and a first reagent piece 22a and a second reagent piece 22b provided in the main body portion 20. Hereinafter, the first reagent piece 22a and the second reagent piece 22b may be simply referred to as "reagent piece 22" unless otherwise distinguished.

In the main body portion 20, the insertion and detachment direction of the blood glucose meter 16 coincides with the longitudinal direction (arrow X direction) of the main body portion 20. One end portion (end portion in arrow X1 direction) of the main body portion 20 is exposed from the blood glucose meter 16 when the main body portion 20 is attached to the blood glucose meter 16 (state of Fig. 1). One end portion of the main body portion 20 is formed in a substantially semicircular shape when viewed from the thickness direction of the main body portion 20 (when viewed from arrow Z direction).

The other end portion (end portion in arrow X2 direction) of the main body portion 20 is accommodated in the blood glucose meter 16 when the main body portion 20 is attached to the blood glucose meter 16 (state of Fig. 1). The other end portion (end portion in arrow X2 direction) of the main body portion 20 is formed in a rectangular shape as viewed from the thickness direction of the main body portion 20 (as viewed from arrow Z direction). That is, the outer shape of the main body portion 20 is a substantially rectangular shape in which one side (side in arrow X1 direction) bulges in an arc shape when viewed from the thickness direction. Note that when the main body portion 20 is attached to the blood glucose meter 16, a region including at least the first reagent piece 22a and the second reagent piece 22b from the other end (end in arrow X2 direction) of the main body portion 20 is accommodated in the blood glucose meter 16.

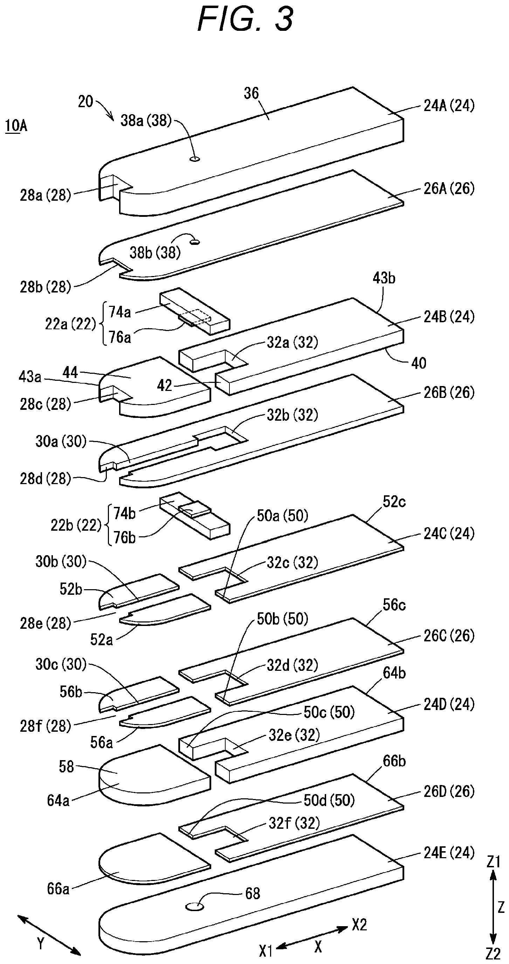

As illustrated in Figs. 2 to 4, the main body portion 20 is formed by sequentially laminating and integrating a first film 24A, a first adhesive layer 26A, a second film 24B, a second adhesive layer 26B, a third film 24C, the third adhesive layer 26C, the fourth film 24D, the fourth adhesive layer 26D, and the fifth film 24E from the upper direction (arrow Z1 direction) toward the lower direction (arrow Z2 direction) in Fig. 3. Hereinafter, the first film 24A, the second film 24B, the third film 24C, the fourth film 24D, and the fifth film 24E may be simply referred to as "film 24" unless otherwise distinguished. In addition, the first adhesive layer 26A, the second adhesive layer 26B, the third adhesive layer 26C, and the fourth adhesive layer 26D may be simply referred to as "adhesive layer 26" unless otherwise distinguished. Each of the film 24 and the adhesive layer 26 exemplified here does not refer to only those composed of a single member. Each of the film 24 and the adhesive layer 26 may be a member made of a plurality of base materials.

The outer edge of each film 24 and each adhesive layer 26 is formed in a substantially rectangular shape having an arc at one end portion when viewed from the arrow Z direction. A space portion is appropriately cut out in each film 24 and each adhesive layer 26.

The main body portion 20 is provided with the inlet portion 28 for taking blood into the main body portion 20, a flow path 30 that guides the blood taken in through the inlet portion 28 onto the reagent piece 22, and a buffer space 32 communicating with the flow path 30.

The inlet portion 28 is provided at one end portion (end portion in arrow X1 direction) of the main body portion 20, which is formed in an arc shape in plan view in the arrow Z direction. One end of the inlet portion 28 in the arrow Z1 direction is open, and the other end in the arrow Z2 direction is covered with the fourth film 24D.

One end (arrow X1 direction) of the flow path 30 is open to the inlet portion 28. The length of the inlet portion 28 in the width direction is longer than the length of the flow path 30 in the width direction. The flow path 30 transfers blood by capillary force. The inlet portion 28, the flow path 30, and the buffer space 32 are formed by laminating space portions formed in the film 24 and the adhesive layer 26.

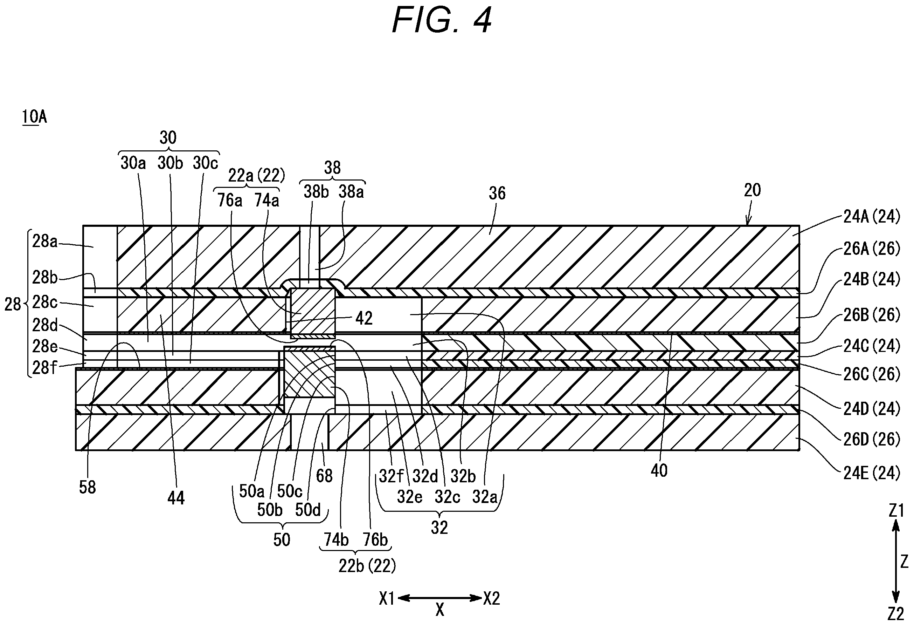

As illustrated in Figs. 3 and 4, the first film 24A is arranged at an end in the arrow Z1 direction. The first film 24A is made of a light shielding member (e.g., black film). First film 24A is made of, for example, polyethylene terephthalate (PET) to which a black pigment is added. That is, the first film 24A includes a light shielding portion 36 that shields a part of the measurement light. The thickness of the first film 24A is preferably set to, for example, 50 um or more and 300 um or less, and more preferably set to 150 um or more and 200 um or less.

A first cutout portion 28a and a first through-hole 38a are formed in the first film 24A. The first cutout portion 28a forms a part of the inlet portion 28. The first cutout portion 28a is formed at an end portion of the first film

24A in the arrow X1 direction. The first cutout portion 28a is formed in a rectangular shape with one side opened in plan view in the arrow Z direction.

The first through-hole 38a is independently provided at a position away from the first cutout portion 28a by a predetermined distance in the arrow X2 direction. The first through-hole 38a penetrates the first film 24A in the arrow Z direction. The first through-hole 38a is located substantially at the center in the width direction (arrow Y direction) of the first film 24A. The first through-hole 38a is formed in a circular shape.

The first adhesive layer 26A is laminated on the first film 24A in the arrow Z2 direction. The first adhesive layer 26A is made of, for example, an acrylic adhesive. The first adhesive layer 26A is a double-sided adhesive tape (adhesive sheet) having an adhesive function on both surfaces. The thickness of the first adhesive layer 26A is preferably set to 15 um or more and 35 um or less, and more preferably set to about 25 µm.

The first adhesive layer 26A is formed in the same shape and the same size as the first film 24A in plan view in the arrow Z direction. Specifically, a second cutout portion 28b and a second through-hole 38b are formed in the first adhesive layer 26A. The second cutout portion 28b forms a part of the inlet portion 28. The second cutout portion 28b communicates with the first cutout portion 28a in the arrow Z2 direction. The second cutout portion 28b is formed in the same size and the same shape as the first cutout portion 28a in plan view in the arrow Z direction.

The second through-hole 38b penetrates the first adhesive layer 26A in the arrow Z direction. The second through-hole 38b communicates with the first through-hole 38a in the arrow Z2 direction. The second through-hole 38b is formed in the same size and the same shape as the first through-hole 38a in plan view in the arrow Z direction.

The first through-hole 38a and the second through-hole 38b communicate with each other to form a first opening 38 for allowing measurement light to pass in the thickness direction of the test strip 10A. By forming the first opening 38 in the first film 24A and the first adhesive layer 26A in this manner, an amount of measurement light necessary for optical detection of a reaction product (measurement target) between a sample and a reagent can reach the detection target through the first opening 38. In addition, since the first film 24A is made of a light shielding member, measurement light can only pass through the first opening 38. As a result, stray light that affects detection accuracy can be reduced.

The first film 24A and the first adhesive layer 26A may be provided with a transparent portion (light guide portion) through which measurement light can pass instead of the first opening 38 (first through-hole 38a and second through-hole 38b).

The second film 24B is laminated on the first adhesive layer 26A in the arrow Z2 direction. The second film 24B is made of, for example, polyester (PE). A first hydrophilic treatment portion 40 (hydrophilic coating) is provided on the surface of the second film 24B in the arrow Z2 direction. The contact angle of the first hydrophilic treatment portion 40 is preferably set to less than 20°. The thickness of the second film 24B is preferably set to 90 um or more and 110 um or less, and more preferably set to about 100 um.

The second film 24B is a first cover layer that covers the flow path 30 from the arrow Z1 direction. In the second film 24B, a third cutout portion 28c, a first buffer hole 32a, and a first reagent placement hole 42 are formed. The third cutout portion 28c forms a part of the inlet portion 28. The third cutout portion 28c is formed at an end portion of the second film 24B in the X1 direction. The third cutout portion 28c communicates with the second cutout portion 28b in the arrow Z2 direction. The third cutout portion 28c is formed in the same size and the same shape as the first and second cutout portions 28a and 28b in plan view in the arrow Z direction.

The first buffer hole 32a forms a part of the buffer space 32. The first buffer hole 32a is a rectangular through-hole penetrating the second film 24B in the thickness direction. The first buffer hole 32a is provided at a position away from the third cutout portion 28c by a predetermined distance in the arrow X2 direction. The first buffer hole 32a is located substantially at the center in the width direction of the second film 24B.

The first reagent placement hole 42 is a space for placing the first reagent piece 22a, and is provided between the third cutout portion 28c and the first buffer hole 32a. The first reagent placement hole 42 penetrates the second film 24B in the thickness direction and extends in a rectangular shape over the entire width (entire length in arrow Y direction) of the second film 24B. The first reagent placement hole 42 communicates with the first buffer hole 32a.

The second film 24B is divided into a first member 43a and a second member 43b by the first reagent placement hole 42. The first member 43a is located in the arrow X1 direction of the second member 43b. The third cutout portion 28c is formed in the first member 43a.

A wall portion 44 of the second film 24B between the third cutout portion 28c and the first reagent placement hole 42 covers the flow path 30 described later from the arrow Z1 direction (see Fig. 4). In the second film 24B, it is possible to provide the first hydrophilic treatment portion 40 only on the surface of the wall portion 44 in the arrow Z2 direction and not provide the first hydrophilic treatment portion 40 in parts other than the wall portion 44.

The second adhesive layer 26B is laminated on the second film 24B in the arrow Z2 direction. The second adhesive layer 26B is made of, for example, an acrylic adhesive. The second adhesive layer 26B is a double-sided adhesive tape (adhesive sheet) having an adhesive function on both surfaces. The second adhesive layer 26B may have adhesive layers on both surfaces of a film-like base material. The thickness of the second adhesive layer 26B is preferably set to 35 um or more and 55 um or less, and more preferably set to about 45 µm.

The second adhesive layer 26B is a first spacer layer for forming the flow path 30. In the second adhesive layer 26B, a fourth cutout portion 28d, a first flow path groove 30a, and a second buffer hole 32b are formed. The fourth cutout portion 28d forms a part of the inlet portion 28. The fourth cutout portion 28d is formed at an end portion of the second adhesive layer 26B in the arrow X1 direction. The fourth cutout portion 28d communicates with the third cutout portion 28c in the arrow Z2 direction. The fourth cutout portion 28d is formed in the same size and the same shape as the first to third cutout portions 28a to 28c in plan view in the arrow Z direction.

The first flow path groove 30a forms a part of the flow path 30. The first flow path groove 30a linearly extends along the longitudinal direction (arrow X direction) of the second adhesive layer 26B. The first flow path groove 30a penetrates the second adhesive layer 26B in the thickness direction. The first flow path groove 30a is located substantially at the center in the width direction of the second adhesive layer 26B.

One end (end in arrow X1 direction and initial end) of the first flow path groove 30a communicates with the fourth cutout portion 28d. The other end (end in arrow X2 direction and terminal end) of the first flow path groove 30a communicates with the second buffer hole 32b. That is, the fourth cutout portion 28d, the first flow path groove 30a, and the second buffer hole 32b form one continuous space.

The first flow path groove 30a is formed to be narrower than the fourth cutout portion 28d. The first flow path groove 30a is covered with the wall portion 44 of the second film 24B from the arrow Z1 direction (see Fig. 4). That is, the wall portion 44 of the second film 24B liquid-tightly blocks the first flow path groove 30a from the first opening 38. The wall portion 44 of the second film 24B is a top surface of the flow path 30 in the arrow Z1 direction.

The second buffer hole 32b forms a part of the buffer space 32. The second buffer hole 32b is a rectangular through-hole penetrating the second adhesive layer 26B in the thickness direction. The second buffer hole 32b is formed at a position facing the first buffer hole 32a. That is, the second buffer hole 32b communicates with the first buffer hole 32a in the arrow Z2 direction. The second buffer hole 32b is formed in the same size and the same shape as the first buffer hole 32a in plan view in the arrow Z direction.

The third film 24C is laminated on the second adhesive layer 26B in the arrow Z2 direction. The third film 24C is made of, for example, polyethylene terephthalate (PET). The thickness of the third film 24C is preferably set to 15 um or more and 35 um or less, and more preferably set to about 25 um. That is, the thickness (length in arrow Z direction) of the third film 24C is thinner than the thickness of the second adhesive layer 26B.

The third film 24C is an intermediate spacer layer for forming the flow path 30. In the third film 24C, a fifth cutout portion 28e, an intermediate flow path groove 30b, a first placement hole 50a, and a third buffer hole 32c are formed. The fifth cutout portion 28e forms a part of the inlet portion 28. The fifth cutout portion 28e is formed at an end portion of the third film 24C in the arrow X1 direction. The fifth cutout portion 28e communicates with the fourth cutout portion 28d in the arrow Z2 direction. The fifth cutout portion 28e is formed in the same shape and the same size as the first to fourth cutout portions 28a to 28d in plan view in the arrow Z direction.

The intermediate flow path groove 30b forms a part of the flow path 30. The intermediate flow path groove 30b extends linearly along the longitudinal direction of the third film 24C. The intermediate flow path groove 30b penetrates the third film 24C in the thickness direction. The intermediate flow path groove 30b is located substantially at the center in the width direction of the third film 24C. One end (end in arrow X1 direction and initial end) of the intermediate flow path groove 30b communicates with the fifth cutout portion 28e. The other end (end in arrow X2 direction and terminal end) of the intermediate flow path groove 30b is connected to the first placement hole 50a.

The intermediate flow path groove 30b is formed to be narrower than the fifth cutout portion 28e. The intermediate flow path groove 30b is formed at a position facing the first flow path groove 30a. That is, the intermediate flow path groove 30b communicates with the first flow path groove 30a in the arrow Z2 direction. The width of the intermediate flow path groove 30b along the arrow Y direction is the same as the width of the first flow path groove 30a along the arrow Y direction. In the arrow X direction, the entire length of the intermediate flow path groove 30b is shorter than the entire length of the first flow path groove 30a (see Fig. 4). The length of the first flow path groove 30a along the arrow Z direction is longer than the length of the intermediate flow path groove 30b along the arrow Z direction.

The first placement hole 50a forms a part of a second reagent placement hole 50 for placing the second reagent piece 22b. The first placement hole 50a is provided between the intermediate flow path groove 30b and the third buffer hole 32c. The first placement hole 50a penetrates the third film 24C in the thickness direction and extends in a rectangular shape over the entire width (entire length in arrow Y direction) of the third film 24C. The first placement hole 50a is provided at an end of the intermediate flow path groove 30b in the arrow X2 direction.

The third film 24C is divided into a first member 52a, a second member 52b, and a third member 52c by the fifth cutout portion 28e, the intermediate flow path groove 30b, and the first placement hole 50a. The first member 52a and the second member 52b are arranged on both sides of the intermediate flow path groove 30b in the arrow Y direction. The third member 52c is arranged in the arrow X2 direction of the first member 52a and the second member 52b with the first placement hole 50a interposed therebetween.

The third buffer hole 32c forms a part of the buffer space 32. The third buffer hole 32c is a rectangular through-hole penetrating the third film 24C in the thickness direction. The third buffer hole 32c is formed at a position facing the second buffer hole 32b. That is, the third buffer hole 32c communicates with the second buffer hole 32b in the arrow Z2 direction. The third buffer hole 32c is formed in the same shape and the same size as the first and second buffer holes 32a and 32b in plan view in the arrow Z direction.

The third adhesive layer 26C is laminated on the third film 24C in the arrow Z2 direction. The third adhesive layer 26C is made of, for example, an acrylic adhesive. The third adhesive layer 26C is a double-sided adhesive tape (adhesive sheet) having an adhesive function on both surfaces. The thickness of the third adhesive layer 26C is preferably set to 15 um or more and 35 um or less, and more preferably set to about 25 um. That is, the thickness of the third adhesive layer 26C is thinner than the thickness of the second adhesive layer 26B. Additionally, the thickness of the third adhesive layer 26C is substantially the same as the thickness of the third film 24C.

The third adhesive layer 26C is a second spacer layer for forming the flow path 30. The third adhesive layer 26C is formed in the same shape and the same size as the third film 24C in plan view in the arrow Z direction. Specifically, in the third adhesive layer 26C, a sixth cutout portion 28f, a second flow path groove 30c, a second placement hole 50b, and a fourth buffer hole 32d are formed. The sixth cutout portion 28f forms a part of the inlet portion 28. The sixth cutout portion 28f communicates with the fifth cutout portion 28e in the arrow Z2 direction. The sixth cutout portion 28f is formed in the same shape and the same size as the first to fifth cutout portions 28a to 28e in plan view in the arrow Z direction.

The second flow path groove 30c forms a part of the flow path 30. The second flow path groove 30c extends linearly along the longitudinal direction of the third adhesive layer 26C. The second flow path groove 30c penetrates the third adhesive layer 26C in the thickness direction. The second flow path groove 30c is located substantially at the center in the width direction of the third adhesive layer 26C. One end (end in arrow X1 direction and initial end) of the second flow path groove 30c communicates with the sixth cutout portion 28f. The other end (end in arrow X2 direction and terminal end) of the second flow path groove 30c is connected to the second placement hole 50b.

The second flow path groove 30c is formed to be narrower than the sixth cutout portion 28f. The second flow path groove 30c is formed at a position facing the intermediate flow path groove 30b. That is, the second flow path groove 30c communicates with the intermediate flow path groove 30b in the arrow Z2 direction. The width of the second flow path groove 30c along the arrow Y direction is the same as the width of the intermediate flow path groove 30b along the arrow Y direction. In the arrow X direction, the entire length of the second flow path groove 30c is equal to the entire length of intermediate flow path groove 30b (see Fig. 4). The length of the first flow path groove 30a along the arrow X direction is longer than the length of the second flow path groove 30c along the arrow X direction. The length of the second flow path groove 30c along the arrow X direction is substantially the same as the length of the intermediate flow path groove 30b along the arrow X direction.

The second placement hole 50b forms a part of the second reagent placement hole 50 for placing the second reagent piece 22b. The second placement hole 50b is provided between the second flow path groove 30c and the fourth buffer hole 32d. The second placement hole 50b communicates with the first placement hole 50a in the arrow Z2 direction.

The third adhesive layer 26C is divided into a first member 56a, a second member 56b, and a third member 56c by the sixth cutout portion 28f, the second flow path groove 30c, and the second placement hole 50b. The first member 56a is located in the arrow Z2 direction of the first member 52a of the third film 24C. The second member 56b is located in the arrow Z2 direction of the second member 52b of the third film 24C. The third member 56c is located in the arrow Z2 direction of the third member 52c of the third film 24C.

The fourth buffer hole 32d forms a part of the buffer space 32. The fourth buffer hole 32d communicates with the third buffer hole 32c in the arrow Z2 direction. The fourth buffer hole 32d is formed in the same shape and the same size as the first to third buffer holes 32a to 32c in plan view in the arrow Z direction.

The fourth film 24D is laminated on the third adhesive layer 26C in the arrow Z2 direction. The fourth film 24D is made of, for example, polyester (PE). A second hydrophilic treatment portion 58 (hydrophilic coating) is provided on the surface of the fourth film 24D in the arrow Z1 direction. The contact angle of the second hydrophilic treatment portion 58 is preferably set to less than 20°. The thickness of the fourth film 24D is preferably set to 80 um or more and 120 um or less, more preferably set to 90 um or more and 110 um or less, and still more preferably set to about 100 µm.

The fourth film 24D is a second cover layer covering the flow path 30 from the arrow Z2 direction. A third placement hole 50c and a fifth buffer hole 32e are formed in the fourth film 24D. The third placement hole 50c forms a part of the second reagent placement hole 50 for placing the second reagent piece 22b. The third placement hole 50c is formed at a position facing the second placement hole 50b. The third placement hole 50c is formed in the same shape and the same size as the first placement hole 50a and the second placement hole 50b when viewed from the arrow Z direction. The third placement hole 50c penetrates the fourth film 24D in the thickness direction and extends over the entire width (entire length in arrow Y direction) of the fourth film 24D.

The fourth film 24D is divided into a first member 64a and a second member 64b by the third placement hole 50c. The first member 64a is located in the arrow X1 direction of the second member 64b. The first member 64a liquid-tightly covers the sixth cutout portion 28f and the second flow path groove 30c from the arrow Z2 direction (see Fig. 4). An end portion of the first member 64a in the arrow X1 direction is formed in a semicircular shape.

The fifth buffer hole 32e forms a part of the buffer space 32. The fifth buffer hole 32e is a rectangular through-hole penetrating the fourth film 24D in the thickness direction. The fifth buffer hole 32e is formed at a position facing the fourth buffer hole 32d. That is, the fifth buffer hole 32e communicates with the fourth buffer hole 32d in the arrow Z2 direction. The fifth buffer hole 32e is formed in the same shape and the same size as the first to fourth buffer holes 32a to 32d in plan view in the arrow Z direction. In the fourth film 24D, it is possible to provide the second hydrophilic treatment portion 58 only on the surface of the first member 64a in the arrow Z1 direction and not provide the second hydrophilic treatment portion 58 in parts other than the first member 64a (second member 64b).

The fourth adhesive layer 26D is laminated on the fourth film 24D in the arrow Z2 direction. The fourth adhesive layer 26D is made of, for example, an acrylic adhesive. The fourth adhesive layer 26D is a double-sided adhesive tape (adhesive sheet) having an adhesive function on both surfaces. The thickness of the fourth adhesive layer 26D is preferably set to 15 um or more and 35 um or less, and more preferably set to about 25 µm.

The fourth adhesive layer 26D is formed in the same shape and the same size as the fourth film 24D in plan view in the arrow Z direction. Specifically, a fourth placement hole 50d and a sixth buffer hole 32f are formed in the fourth adhesive layer 26D. The fourth placement hole 50d forms a part of the second reagent placement hole 50 for placing the second reagent piece 22b. The fourth placement hole 50d communicates with the third placement hole 50c in the arrow Z2 direction. The fourth placement hole 50d is formed in the same shape and the same size as the third placement hole 50c in plan view in the arrow Z direction. The first placement hole 50a, the second placement hole 50b, the third placement hole 50c, and the fourth placement hole 50d communicate with each other to form one second reagent placement hole 50.

The fourth adhesive layer 26D is divided into a first member 66a and a second member 66b by the fourth placement hole 50d. The first member 66a is located in the arrow Z2 direction of the first member 64a of the fourth film 24D. The second member 66b is located in the arrow Z2 direction of the second member 64b of the fourth film 24D.

The sixth buffer hole 32f forms a part of the buffer space 32. The sixth buffer hole 32f communicates with the fifth buffer hole 32e in the arrow Z2 direction. The sixth buffer hole 32f is formed in the same shape and the same size as the first to fifth buffer holes 32a to 32e in plan view in the arrow Z direction.

The fifth film 24E may be further laminated on the fourth adhesive layer 26D in the arrow Z2 direction. The fifth film 24E is arranged at the end in the thickness direction (end in arrow Z2 direction) of the test strip 10A. The fifth film 24E forms one surface of the test strip 10A. The fifth film 24E liquid-tightly covers the sixth buffer hole 32f from the arrow Z2 direction (see Fig. 4). A second opening 68 is formed in the fifth film 24E.

The second opening 68 is a circular through-hole that transmits measurement light in the thickness direction of the test strip 10A. The second opening 68 is positioned in the arrow Z2 direction of the first opening 38. The diameter of the second opening 68 is larger than the diameter of the first opening 38. In other words, the second opening 68 is provided such that the entire first opening 38 is located inside the second opening 68 when viewed from the arrow Z direction. Note that the fifth film 24E may be provided with a transparent portion (light guide portion) through which measurement light can pass instead of the second opening 68.

In the main body portion 20 configured as described above, the inlet portion 28 is formed by the first to sixth cutout portions 28a to 28f. The flow path 30 is formed by the first flow path groove 30a, the intermediate flow path groove 30b, and the second flow path groove 30c. The buffer space 32 is formed by the first to sixth buffer holes 32a to 32f.

In Fig. 4, the initial end of the flow path 30 communicates with the inlet portion 28. The terminal end of the flow path 30 (terminal end of first flow path groove 30a) communicates with the buffer space 32. The first reagent piece 22a and the second reagent piece 22b for analyte detection are placed at any position between the initial end and the terminal end of the flow path 30. That is, the buffer space 32 exists downstream of the first reagent piece 22a and the second reagent piece 22b in the flow path 30.

As illustrated in Figs. 3 to 5, the first reagent piece 22a includes a first support base 74a and a first reagent portion 76a provided on the first support base 74a. In plan view in the arrow Z direction, the first support base 74a is formed in a rectangular shape. The first reagent portion 76a is located substantially at the center in the longitudinal direction (arrow Y direction) of the first support base 74a. The surface of the first support base 74a in the arrow Z1 direction is bonded to the surface of the first adhesive layer 26A in the arrow Z2 direction. The surfaces of the first support base 74a in the arrow Z2 direction on both sides of the first reagent portion 76a in the longitudinal direction are bonded to the surface of the second adhesive layer 26B in the arrow Z1 direction such that the first reagent portion 76a is positioned in the first flow path groove 30a. In this case, a first gap Sa is formed between an inner surface (first inner surface 41) of the first reagent placement hole 42 on the upstream side of the flow path 30 and the first support base 74a. The length of the first gap Sa along the arrow X direction is more than 0 mm and 0.1 mm or less. The arrow Z1 side of the first gap Sa is sealed by the first adhesive layer 26A in order to prevent blood leakage to the first opening 38. At this time, the first adhesive layer 26A adheres the first film 24A and the first support base 74a in a slightly compressed state. The first adhesive layer 26A can prevent blood from reaching the first opening 38 even if blood leaks into the first gap Sa.

The first support base 74a extends long in the lateral direction (width direction, i.e., arrow Y direction) of the test strip 10A. That is, the first support base 74a is formed in a rectangular shape where the length of the first support base 74a in the lateral direction of the test strip 10A is shorter than the length of the first support base 74a in the longitudinal direction (arrow X direction) of the test strip 10A. As the first support base 74a, a film material having transparency can be used.

The first reagent portion 76a is obtained by supporting a reagent that reacts with a sample on at least a part of the flow path 30. The first reagent portion 76a is applied to the first support base 74a without blocking the flow path 30. Various polymers and coating compositions may be further arranged on the first support base 74a depending on the properties of the reagent and the measurement system.

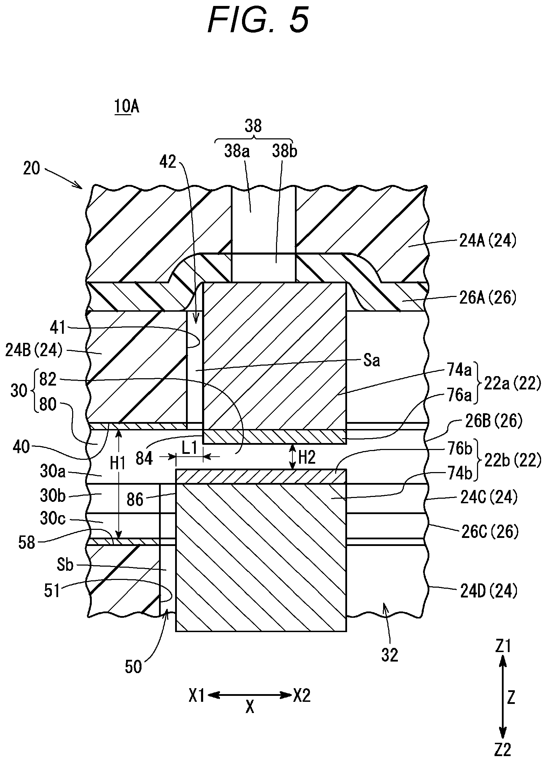

In plan view in the arrow Z direction, the first reagent portion 76a is formed in a quadrangular shape (see Fig. 3). As illustrated in Fig. 5, in the arrow X direction, the length of the first reagent portion 76a is the same as the length of the first support base 74a. The length of the first reagent portion 76a in the arrow X direction is set to, for example, 1.0 mm or more and 2.0 mm or less.

The second reagent piece 22b includes a second support base 74b and a second reagent portion 76b provided on the second support base 74b. The second reagent piece 22b is basically configured similarly to the first reagent piece 22a. Note, however, that the length of the second reagent piece 22b in the arrow X direction is longer than the length of the first reagent piece 22a in the arrow X direction. The second reagent portion 76b is located substantially at the center in the longitudinal direction (arrow Y direction) of the second support base 74b. Both sides of the second reagent portion 76b in the longitudinal direction of the second support base 74b are bonded to the surface of the second adhesive layer 26B in the arrow Z2 direction such that the second reagent portion 76b is positioned in the first flow path groove 30a. In this case, a second gap Sb is formed between an inner surface (second inner surface 51) of the second reagent placement hole 50 on the upstream side of the flow path 30 and the second support base 74b. The length of the second gap Sb in the arrow X direction is more than 0 mm and 0.1 mm or less.

The second reagent portion 76b is configured similarly to the first reagent portion 76a. Therefore, detailed description of the configuration of the second reagent portion 76b is omitted. Note that the reagent components contained in the first reagent portion 76a and the second reagent portion 76b may be the same or different from each other. The second reagent portion 76b is formed in a quadrangular shape (see Fig. 3). In the arrow X direction, the length of the second reagent portion 76b is the same as the length of the second support base 74b. The length of the second reagent portion 76b in the arrow X direction is set to, for example, 1.0 mm or more and 2.0 mm or less.

The length of the second reagent portion 76b in the arrow X direction is longer than the length of the first reagent portion 76a in the arrow X direction. The first reagent portion 76a and the second reagent portion 76b are arranged in the flow path 30 so as to face each other in the vertical direction. In the flow direction of blood in the flow path 30, the upstream end of the second reagent portion 76b is offset in the arrow X1 direction from the upstream end of the first reagent portion 76a. Note that in the flow direction of blood in the flow path 30, the downstream end of the second reagent portion 76b is at the same position as the downstream end of the first reagent portion 76a. Note, however, that the downstream end of the second reagent portion 76b and the downstream end of the first reagent portion 76a may be arranged at the same position or may be offset from each other in the flow direction of blood (arrow X direction) in the flow path 30.

In the test strip 10A configured as described above, the flow path 30 includes an introduction flow path 80 and a reaction flow path 82. The introduction flow path 80 guides blood (sample) introduced into the inlet portion 28 to the reaction flow path 82. The introduction flow path 80 extends from the inlet portion 28 to the first reagent portion 76a. The reaction flow path 82 is a portion between the first reagent portion 76a and the second reagent portion 76b. Note that the second reagent portion 76b is arranged below the first reagent portion 76a. The reaction flow path 82 (first reagent portion 76a and second reagent portion 76b) overlaps the first opening 38 when viewed from the arrow Z direction (see Fig. 4). Therefore, measurement light of the blood glucose meter 16 is emitted toward the reaction flow path 82.

The length of the introduction flow path 80 in the arrow Z direction (first flow path height H1) is longer (higher) than the length of the reaction flow path 82 in the arrow Z direction (second flow path height H2). The second flow path height H2 is preferably set to 20 um or more and 40 um or less, and is set to 30 µm, for example. Note, however, that the second flow path height H2 can be appropriately set.

The flow path 30 includes a first step portion 84 and a second step portion 86. The first step portion 84 is located at the upstream end (end in arrow X1 direction) of the flow path 30 in the first reagent portion 76a. The second step portion 86 is located at the upstream end (end in arrow X1 direction) of the flow path 30 in the second reagent portion 76b. The height from the bottom surface (second hydrophilic treatment portion 58) of the flow path 30 to the upper surface of the second reagent portion 76b (height of second step portion 86) is higher than the height from the upper surface (first hydrophilic treatment portion 40) of the flow path 30 to the lower surface of the first reagent portion 76a (height of first step portion 84). In the flow direction of blood in the flow path 30, the first step portion 84 and the second step portion 86 are offset from each other. Specifically, the second step portion 86 is located upstream of the first step portion 84 in the flow path 30. An offset length L1 between the first step portion 84 and the second step portion 86 is 0.1 mm or more. The offset length L1 is preferably 0.2 mm or more and 0.8 mm or less.

The second step portion 86 is located upstream (arrow X1 direction) of the first gap Sa in the flow path 30. Therefore, the second reagent portion 76b covers the first gap Sa from the arrow Z2 direction. Additionally, the first hydrophilic treatment portion 40 covers the second gap Sb from the arrow Z1 direction. In the flow direction of blood in the flow path 30, the first gap Sa and the second gap Sb are offset so as not to face each other across the flow path 30. In other words, the second gap Sb is offset to the upstream side of the flow path 30 with respect to the first gap Sa. Moreover, the second inner surface 51 is located upstream of the first inner surface 41 in the flow path 30.

The plurality of films 24 is not limited to the examples made of the above-described materials. The plurality of films 24 can be made of resin materials such as polyethylene terephthalate (PET), polymethyl methacrylate (PMMA), polyester, polycarbonate, polystyrene, polypropylene, acrylonitrile-butadiene-styrene copolymer (ABS), cycloolefin polymer (COP), and cyclic olefin copolymer (COC). In a case where the component measurement device 14 is of a type that detects measurement light (transmitted light) transmitted through the detection target, the path of the measurement light is made of a material having transparency. As the film 24, a pigment may be mixed depending on the purpose, and when the film 24 is used as a light shielding member, a resin material containing carbon black can be used. Note that based on the measurement method of JIS K7605;1976 (abolished standard), the light shielding ratio of the light shielding member is preferably 90% or more, and a black film member having a light shielding ratio of 99% or more can be suitably used.

In the test strip 10A, the flow path 30 is not limited to the example formed in three layers of the first spacer layer, the intermediate spacer layer, and the second spacer layer, and may be formed over four or more layers.

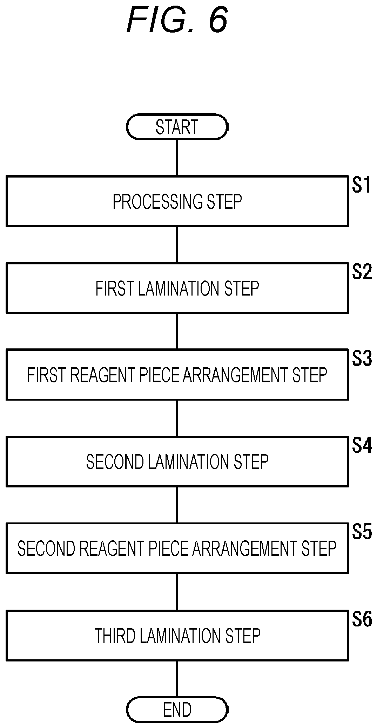

The above-described test strip 10A is manufactured by cutting and laminating a plurality of films 24 subjected to an appropriate surface treatment and a plurality of adhesive sheets. Specifically, as illustrated in Fig. 6, the method of manufacturing the test strip 10A includes a processing step, a first lamination step, a first reagent piece placement step, a second lamination step, a second reagent piece placement step, and a third lamination step.

In the processing step (step S1), the first to fifth films 24A to 24E are formed by processing (punching) the film 24 with a cutting tool, and the first to fourth adhesive layers 26A to 26D are formed by processing (punching) the adhesive sheet with the cutting tool.

In the first lamination step (step S2), the first film 24A, the first adhesive layer 26A, and the second film 24B are laminated and bonded in this order.

In the first reagent piece placement step (step S3), the first reagent piece 22a is placed in the first reagent placement hole 42. At this time, both sides of the first reagent portion 76a in the first support base 74a are bonded to the surface of the first adhesive layer 26A in the arrow Z2 direction. As a result, the first reagent piece 22a is fixed to the first adhesive layer 26A.

In the second lamination step (step S4), the second adhesive layer 26B, the third film 24C, the third adhesive layer 26C, the fourth film 24D, and the fourth adhesive layer 26D are bonded to the second film 24B while being laminated in this order.

In the second reagent piece placement step (step S5), the second reagent piece 22b is inserted from the fourth placement hole 50d into the second reagent placement hole 50. At this time, both sides of the second reagent portion 76b in the second support base 74b are bonded to the surface of the second adhesive layer 26B in the arrow Z2 direction. As a result, the second reagent piece 22b is fixed to the second adhesive layer 26B.

In the third lamination step (step S6), the fifth film 24E is bonded to the fourth adhesive layer 26D. Thus, the test strip 10A is manufactured.

The method of manufacturing the test strip 10A is not limited to the method described above.

Next, the blood glucose meter 16 to which the test strip 10A is attached will be described. As illustrated in Fig. 1, the blood glucose meter 16 is configured as a reusable type capable of repeatedly measuring the blood glucose level. A housing 90 of the blood glucose meter 16 has a box portion 94 having a size that allows the user to easily grip and operate the blood glucose meter and accommodating a control unit 92 of the blood glucose meter 16 therein, and a cylindrical photometric unit 96 protruding from the box portion 94 and accommodating the measurement unit 18 of the optical system therein.

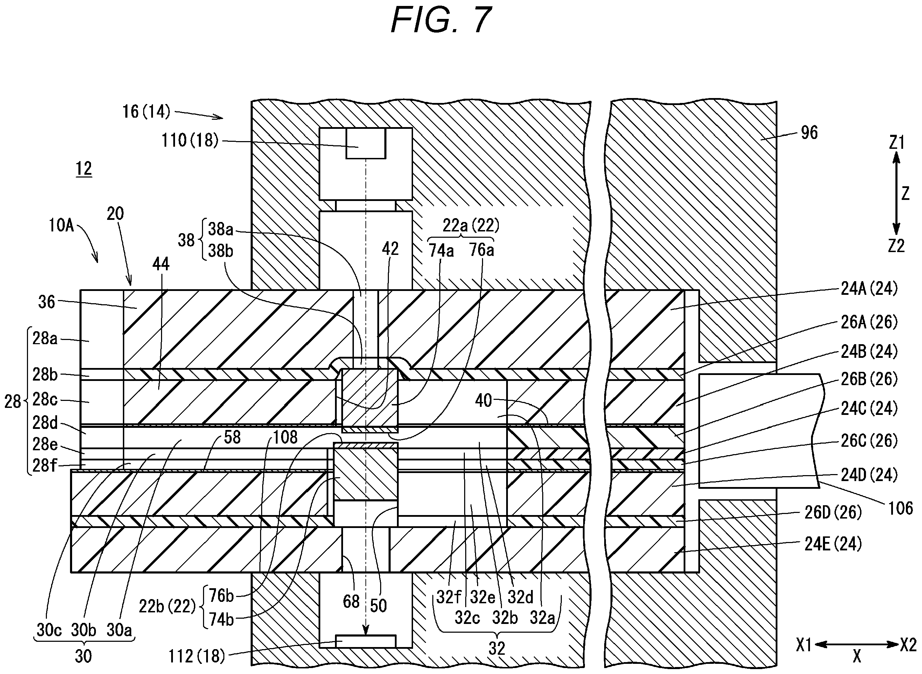

A power button 98, an operation button 100, and a display 102 are provided on an upper surface of the box portion 94. An eject lever 104 as an operation unit for detaching the test strip 10A after use is provided on an upper surface of the photometric unit 96. The eject lever 104 is provided to be movable along the extending direction of the photometric unit 96, and is connected to an eject pin 106 (see Fig. 7) provided in the photometric unit 96.

As illustrated in Fig. 7, the photometric unit 96 is provided with an insertion hole 108 into which the test strip 10A is inserted. The measurement unit 18 optically detects glucose in blood. The measurement unit 18 includes a light emitting unit 110 and a light receiving unit 112. The light emitting unit 110 and the light receiving unit 112 are arranged to face each other with the insertion hole 108 interposed therebetween.

As the light emitting unit 110, an LED, an organic EL, a laser diode, or the like is used. In a state where the test strip 10A is attached to the insertion hole 108, the light emitting unit 110 emits light having a predetermined wavelength toward the first opening 38 of the test strip 10A. As the light receiving unit 112, for example, a photodiode is used. The light receiving unit 112 receives the light transmitted through the test strip 10A (first reagent portion 76a and second reagent portion 76b).

The control unit 92 of the blood glucose meter 16 includes a control circuit (computer) having a converter, a processor, a memory, and an input/output interface (not illustrated). For example, the control unit 92 drives the measurement unit 18 under the operation of the user to calculate the blood glucose level based on a signal corresponding to the amount (or concentration) of glucose in the blood. The calculated blood glucose level is displayed on the display 102.

Next, the measurement of the blood glucose level using the test strip 10A according to the present embodiment will be described.

As illustrated in Fig. 7, the user inserts the test strip 10A into the insertion hole 108 of the blood glucose meter 16, and positions the first reagent portion 76a and the second reagent portion 76b of the test strip 10A between the light emitting unit 110 and the light receiving unit 112. Next, the user attaches a small amount of blood to the inlet portion 28 of the test strip 10A. The blood flows through the introduction flow path 80 toward the reagent portion by capillary force. Specifically, on the inner surface of the introduction flow path 80, the blood is pulled in the direction toward the reagent portion (arrow X2 direction) by the surface tension of the first hydrophilic treatment portion 40 provided on the surface of the second film 24B and the surface tension of the second hydrophilic treatment portion 58 provided on the surface of the fourth film 24D.

When the blood reaches the second gap Sb, the action of the surface tension by the fourth film 24D is lost, so that the surface tension acting on the blood is reduced. However, at this time, since the first hydrophilic treatment portion 40 on the second film 24B exists at a position facing the second gap Sb, tension toward the first hydrophilic treatment portion 40 acts on the blood. Therefore, the blood flows through the second gap Sb and over the second step portion 86 to the upper space of the second reagent portion 76b.

Next, when the blood reaches the first gap Sa, the surface tension on the second film 24B is lost on the first gap Sa, so that the surface tension acting on the blood is reduced. However, at this time, since the second reagent portion 76b is positioned so as to face the first gap Sa, tension toward the second reagent portion 76b dominantly acts on the blood. Therefore, the blood can flow through the first gap Sa and over the first step portion 84, to flow between the first reagent portion 76a and the second reagent portion 76b (reaction flow path 82) in the arrow X2 direction. The first reagent portion 76a is placed on the first step portion 84. A space between the first reagent portion 76a and the second reagent portion 76b (reaction flow path 82) is narrower than the introduction flow path 80. Therefore, inflow into the reaction flow path 82 is even more difficult due to the increased shear stress effect of the blood.

However, in the reaction flow path 82, since the surface tension by the second reagent portion 76b and the first reagent portion 76a acts on the blood, the blood reaches the downstream end of the reaction flow path 82. As a result, the first reagent portion 76a diffuses while being dissolved in the blood in the reaction flow path 82, and flows toward the second reagent portion 76b (arrow Z2 direction). Similarly, the second reagent portion 76b is also dissolved and diffused in the blood in the reaction flow path 82. The blood in which the reagent components of the first reagent portion 76a and the second reagent portion 76b are dissolved flows to the downstream end of the second reagent portion 76b and the downstream end of the first reagent portion 76a (arrow X2 direction).

In the present embodiment, the diffusion distance of each of the reagent component in the first reagent portion 76a and the reagent component in the second reagent portion 76b is half the second flow path height H2 of the reaction flow path 82. Therefore, the diffusion time of the reagent component into the blood is shortened, and the measurement standby time can be shortened. In addition, the test strip 10A can be made such that the blood and each reagent can react even under a low temperature or a condition where the amount of red blood cells is large and the blood is difficult to flow like blood of high hematocrit blood.

At this time, glucose in the blood reacts with the reagent component. Note that in the present embodiment, the reagent component contains a hemolytic agent. Therefore, the reagent component causes hemolysis of red blood cells in blood. As a result, the blood in the reaction flow path 82 becomes transparent and scattering is reduced, so that the accuracy of optical measurement is improved. In addition, glucose in red blood cells can also react with the reagent component, so that the measurement accuracy of the blood glucose level is improved.

Next, the user operates the operation button 100 of the blood glucose meter 16 to start the measurement of the blood glucose level. Then, measurement light emitted from the light emitting unit 110 passes through the first opening 38, the wall portion 44 of the second film 24B, the first flow path groove 30a, the first reagent piece 22a, the reaction flow path 82, the second reagent piece 22b, and the second opening 68, and is received by the light receiving unit 112. The control unit 92 of the blood glucose meter 16 calculates the blood glucose level based on the output signal from the light receiving unit 112, and displays the blood glucose level on the display 102. As a result, the measurement of the blood glucose level ends.

The present embodiment has the following effects.

According to the present embodiment, the first reagent portion 76a and the second reagent portion 76b are provided in the flow path 30 so as to face each other while being separated from each other. Therefore, when the blood introduced from the inlet portion 28 arrives between the first reagent portion 76a and the second reagent portion 76b (reaction flow path 82), the reagent component of the first reagent portion 76a and the reagent component of the second reagent portion 76b diffuse into the blood. At this time, the diffusion distance of the reagent component is half the distance between the first reagent portion 76a and the second reagent portion 76b (second flow path height H2). Therefore, the diffusion time (measurement standby time) of the reagent component into the blood can be made relatively short.

In addition, the first step portion 84 and the second step portion 86 are offset from each other in the flow direction of blood in the flow path 30. Specifically, the second reagent portion 76b is located below the first reagent portion 76a, and the second step portion 86 is located upstream of the first step portion 84 in the flow path 30. In this case, the blood passes through the first step portion 84 after passing through the second step portion 86. As a result, the flow path resistance can be dispersed in the flow direction of blood in the flow path 30 as compared with a case where the first step portion 84 and the second step portion 86 are provided at the same position in the flow direction of blood in the flow path 30. Therefore, the blood introduced from the inlet portion 28 can be smoothly guided to the reaction flow path 82. Therefore, the measurement standby time can be shortened even under a condition where the blood is difficult to flow.

The offset length L1 is 0.2 mm or more and 0.8 mm or less.

According to such a configuration, the blood introduced from the inlet portion 28 can be more smoothly guided to the reaction flow path 82.

A hydrophilic treatment portion is provided on the inner surface of the flow path 30.

According to such a configuration, the blood introduced from the inlet portion 28 can be more smoothly guided to the reaction flow path 82.

The test strip 10A includes the first reagent piece 22a, the second reagent piece 22b, the first reagent placement hole 42, and the second reagent placement hole 50. The first reagent piece 22a is formed by providing the first reagent portion 76a on the first support base 74a. The second reagent piece 22b is formed by providing the second reagent portion 76b on the second support base 74b. The first reagent piece 22a is placed in the first reagent placement hole 42. The second reagent piece 22b is placed in the second reagent placement hole 50. The first gap Sa is formed between an inner surface (first inner surface 41) of the first reagent placement hole 42 on the upstream side of the flow path 30 and the first reagent piece 22a. The second gap Sb is formed between an inner surface (second inner surface 51) of the second reagent placement hole 50 on the upstream side of the flow path 30 and the second reagent piece 22b. In the flow direction of blood in the flow path 30, the first gap Sa and the second gap Sb are offset so as not to face each other.

According to such a configuration, it is possible to curb an excessive decrease in surface tension acting on the blood when the blood introduced from the inlet portion 28 is guided to the reaction flow path 82.

### (Second embodiment)

Next, a test strip 10B according to a second embodiment of the present invention will be described. In the test strip 10B according to the present embodiment, the same components as those of the above-described test strip 10A are denoted by the same reference numerals, and detailed description thereof is omitted.

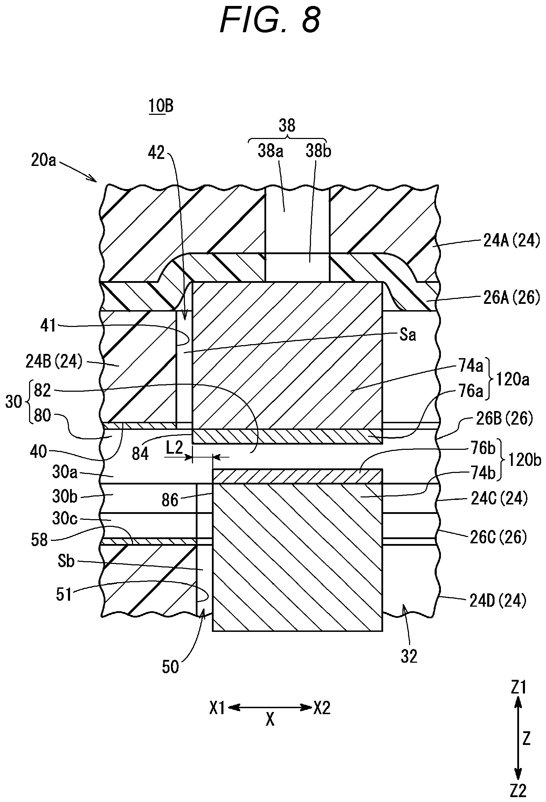

As illustrated in Fig. 8, the test strip 10B includes a main body portion 20a, a first reagent piece 120a, and a second reagent piece 120b. The main body portion 20a is configured similarly to the main body portion 20 described above except that a second inner surface 51 on the upstream side of a second reagent placement hole 50 is located downstream of a first inner surface 41 on the upstream side of a first reagent placement hole 42 in a flow path 30. The first reagent piece 120a and the second reagent piece 120b are configured similarly to the first reagent piece 22a and the second reagent piece 22b described above. Note, however, that the length of the second reagent piece 120b in the arrow X direction is shorter than the length of the first reagent piece 120a in the arrow X direction.

In the test strip 10B, a second step portion 86 is located downstream of a first step portion 84 in the flow path 30. An offset length L2 between the first step portion 84 and the second step portion 86 is 0.1 mm or more. The offset length L2 is preferably 0.2 mm or more and 0.8 mm or less.

The first step portion 84 is located upstream (arrow X1 direction) of a second gap Sb in the flow path 30. Therefore, the first reagent portion 76a covers the arrow Z1 side of the second gap Sb. In addition, a second hydrophilic treatment portion 58 covers the arrow Z2 side of a first gap Sa. In the flow direction of blood in the flow path 30, the first gap Sa and the second gap Sb are offset so as not to face each other. In other words, the second gap Sb is offset to the downstream side of the flow path 30 with respect to the first gap Sa.

According to such a test strip 10B, an effect similar to that of the test strip 10A described above is obtained.

Next, a test performed to confirm the effect of the present invention will be described.

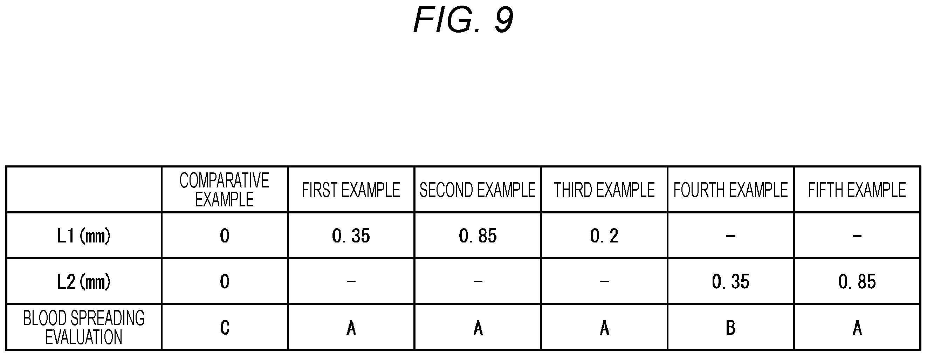

In the present test, as illustrated in Fig. 9, six each of a test strip 200 according to a comparative example, a test strip 10A according to a first example, a test strip 10A according to a second example, a test strip 10A according to a third example, a test strip 10B according to a fourth example, and a test strip 10B according to a fifth example were prepared.

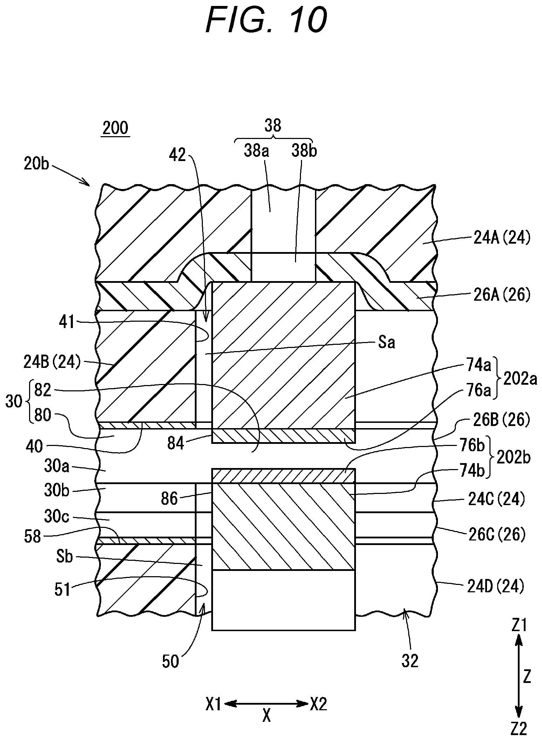

As illustrated in Fig. 10, the test strip 200 according to the comparative example includes a main body portion 20b, a first reagent piece 202a, and a second reagent piece 202b. The main body portion 20b is configured similarly to the main body portion 20 described above except that a second inner surface 51 and a first inner surface 41 are at the same position in the flow direction of blood (arrow X direction) in the flow path 30.

The first reagent piece 202a and the second reagent piece 202b are configured similarly to the first reagent piece 22a and the second reagent piece 22b described above. Note, however, that the length of the second reagent piece 202b in the arrow X direction is the same as the length of the first reagent piece 202a in the arrow X direction. In the test strip 200, a first step portion 84 and a second step portion 86 are at the same position in the flow direction of blood in the flow path 30. A first gap Sa and a second gap Sb are positioned so as to face each other.

The first to third examples are the test strip 10A according to the first embodiment. In the first example, the offset length L1 is set to 0.35 mm. In the second example, the offset length L1 is set to 0.85 mm. In the third example, the offset length L1 is set to 0.2 mm. The fourth example and the fifth example are the test strips 10B according to the second embodiment. In the fourth example, the offset length L2 is set to 0.35 mm. In the fifth example, the offset length L2 is set to 0.85 mm.

### [Experimental method]

A blood circulation test (blood spreading evaluation) was performed on each of the test strips 200, 10A, and 10B. Specifically, in each of the test strips 200, 10A, and 10B, blood was introduced into the inlet portion 28 under an environment of 25°C, and it was confirmed whether or not the blood flowed to the downstream end of the reaction flow path 82 (whether or not the blood spread). Note that the hematocrit value (Ht) of the used blood is 60%.

The blood spreading was evaluated in three stages of A, B, and C. The blood spreading evaluation was A when blood spread in all of the six test strips 200, 10A, and 10B. The blood spreading evaluation was B when blood spread in five of the six test strips 200, 10A, and 10B and blood did not spread in one. The blood spreading evaluation was C when blood spread in one of the six test strips 200, 10A, 10B and blood did not spread in five.

### [Results]

As illustrated in Fig. 9, the blood spreading evaluation of each of the first to third examples and the fifth example was A. The blood spreading evaluation of the fourth example was B. The blood spreading evaluation of the comparative example was C. From the above, the result was obtained that blood can be smoothly guided to the reaction flow path 82 by offsetting the first step portion 84 and the second step portion 86 from each other in the flow direction of blood in the flow path 30. In addition, a result was obtained that the blood spreading evaluation was good when the offset lengths L1 and L2 were 0.2 mm or more.

In the test strips 10A and 10B described above, the number of laminated layers of each of the film 24 and the adhesive layer 26 can be appropriately set. In addition, the test strips 10A and 10B may be configured such that the first reagent portion 76a and the second reagent portion 76b are directly applied to the inner surface of the flow path 30 without forming the first reagent placement hole 42 and the second reagent placement hole 50 in the main body portions 20 and 20a. In this case, the test strips 10A and 10B do not have the first gap Sa and the second gap Sb, but have the first step portion 84 and the second step portion 86.

The present invention is not limited to the embodiments described above and may be modified in various manners without departing from the gist of the present invention.

The above-described embodiments are summarized as follows.

The above embodiment discloses a test strip (10A, 10B) having a flow path (30) for circulating a sample introduced from an inlet portion (28) by capillary force, the test strip including a first reagent portion (76a) and a second reagent portion (76b) provided in the flow path so as to face each other while being separated from each other, in which the flow path includes a first step portion (84) located at an upstream end of the flow path in the first reagent portion and a second step portion (86) located at an upstream end of the flow path in the second reagent portion, and the first step portion and the second step portion are offset from each other in a flow direction of the sample in the flow path.

In the test strip, an offset length (L1, L2) between the first step portion and the second step portion may be 0.1 mm or more.

In the test strip, the offset length may be 0.2 mm or more.

In the test strip, the second reagent portion may be located below the first reagent portion, and the second step portion may be located upstream of the first step portion in the flow path.

In the test strip, the second reagent portion may be located below the first reagent portion, and the second step portion may be located downstream of the first step portion in the flow path.

In the test strip, a hydrophilic treatment portion (40, 58) may be provided on an inner surface of the flow path.

In the test strip, the test strip may include a first reagent piece (22a, 120a) formed by providing the first reagent portion on a first support base (74a), a second reagent piece (22b, 120b) formed by providing the second reagent portion on a second support base (74b), a first reagent placement hole (42) in which the first reagent piece is placed, and a second reagent placement hole (50) in which the second reagent piece is placed. A first gap (Sa) may be formed between an inner surface (41) on the upstream side of the flow path in the first reagent placement hole and the first reagent piece, a second gap (Sb) may be formed between an inner surface (51) on the upstream side of the flow path in the second reagent placement hole and the second reagent piece, and the first gap and the second gap may be offset so as not to face each other.

### Reference Signs List

10A, 10B Test strip
20, 20a, 20b Main body portion
22a, 120a First reagent piece
22b, 120b Second reagent piece
28 Inlet portion
30 Flow path
40 First hydrophilic treatment portion (hydrophilic treatment portion)
41 First inner surface (inner surface)
42 First reagent placement hole
50 Second reagent placement hole
51 Second inner surface (inner surface)
58 Second hydrophilic treatment portion (hydrophilic treatment portion)
74a First support base
74b Second support base
76a First reagent portion
76b Second reagent portion
84 First step portion
86 Second step portion
L1, L2 Offset length
Sa First gap
Sb Second gap

## Claims

1. A test strip having a flow path for circulating a sample introduced from an inlet portion by capillary force, the test strip comprising a first reagent portion and a second reagent portion provided in the flow path so as to face each other while being separated from each other, wherein
the flow path includes
a first step portion located at an upstream end of the flow path in the first reagent portion and
a second step portion located at an upstream end of the flow path in the second reagent portion, and
the first step portion and the second step portion are offset from each other in a flow direction of the sample in the flow path.

2. The test strip according to claim 1, wherein
an offset length between the first step portion and the second step portion is 0.1 mm or more.

3. The test strip according to claim 2, wherein
the offset length is 0.2 mm or more.

4. The test strip according to any one of claims 1 to 3, wherein
the second reagent portion is located below the first reagent portion, and
the second step portion is located upstream of the first step portion in the flow path.

5. The test strip according to any one of claims 1 to 3, wherein
the second reagent portion is located below the first reagent portion, and
the second step portion is located downstream of the first step portion in the flow path.

6. The test strip according to any one of claims 1 to 5, wherein
a hydrophilic treatment portion is provided on an inner surface of the flow path.

7. The test strip according to any one of claims 1 to 6 comprising:
a first reagent piece formed by providing the first reagent portion on a first support base;
a second reagent piece formed by providing the second reagent portion on a second support base;
a first reagent placement hole in which the first reagent piece is placed; and
a second reagent placement hole in which the second reagent piece is placed, wherein:
a first gap is formed between an inner surface on the upstream side of the flow path in the first reagent placement hole and the first reagent piece;
a second gap is formed between an inner surface on the upstream side of the flow path in the second reagent placement hole and the second reagent piece; and
the first gap and the second gap are offset so as not to face each other in the flow direction.
